# EUROPEAN PATENT APPLICATION

(11) **EP 4 850 590 A1**
(43) Date of publication of application: **09.12.2026**
(21) Application number: 25748939.3
(22) Date of filing: 31.01.2025
(51) Int. Cl.: A61B 3/10

(54) **INFORMATION PROCESSING SYSTEM, INFORMATION PROCESSING METHOD, AND PROGRAM**

(30) Priority: 31.01.2024 JP 2024012462
(71) Applicant: Innojin, Inc., Tokyo 113-0033 (JP); Juntendo Educational Foundation, Tokyo 113-8421 (JP)
(72) Inventor: INOMATA, Takenori, Tokyo 113-0033 (JP); OKUMURA, Yuichi, Tokyo 113-0033 (JP)
(74) Representative: Hasegawa, Kan
(86) International application number: PCT/JP2025/003170
(87) International publication number: WO 2025/164760

(57) **Abstract**

An information processing system includes an image acquisition unit configured to acquire a captured image of an eye of a subject, and an anterior chamber depth estimation unit configured to input the acquired captured image to a trained model trained by machine learning using images of eyes and anterior chamber depths as training data, thereby estimating an anterior chamber depth of the subject. Thus, a condition of the subject can be readily estimated.

## Description

### TECHNICAL FIELD

The present invention relates to an information processing system, an information processing method, and a program.

### BACKGROUND OF THE INVENTION

Patent Document 1 discloses using results of spectral analysis of an image of a subject's eye to assist in diagnosing cataract.

### PRIOR ART DOCUMENT

### PATENT DOCUMENTS

[Patent Document 1] Japanese Unexamined Patent Application Publication No. 2002-224041

### SUMMARY OF THE INVENTION

### PROBLEM TO BE SOLVED BY THE INVENTION

However, Patent Document 1 requires time and effort to set the illumination light to be directed into the eye.

The present invention has been made in view of such circumstances, and an object thereof is to provide a technique capable of readily estimating a condition of a subject.

### MEANS FOR SOLVING THE PROBLEMS

A principal aspect of the present invention for solving the above problem is an information processing system comprising: an image acquisition unit configured to acquire a captured image of an eye of a subject; and an anterior chamber depth estimation unit configured to input the acquired captured image to a trained model trained by machine learning using images of eyes and anterior chamber depths as training data, thereby estimating an anterior chamber depth of the subject.

Other problems disclosed in the present application and means for solving the problems will become apparent from the embodiments of the invention and the drawings.

### EFFECTS OF THE INVENTION

According to the present invention, a condition of a subject can be readily estimated.

### BRIEF DESCRIPTION OF DRAWINGS

FIG. 1 is a diagram illustrating an example of an overall configuration of an information processing system.
FIG. 2 is a diagram illustrating an example of a hardware configuration of a subject terminal 1.
FIG. 3 is a diagram illustrating an example of a software configuration of the subject terminal 1.
FIG. 4 is a diagram illustrating an example of a hardware configuration of a management server 2.
FIG. 5 is a diagram illustrating an example of a software configuration of the management server 2.
FIG. 6 is a diagram for explaining an operation of the information processing system.
FIG. 7 is a diagram illustrating an example of a software configuration of the management server 2 according to a second embodiment.
FIG. 8 is a diagram illustrating an example of a software configuration of the management server 2 according to a third embodiment.

### DETAILED DESCRIPTION OF THE INVENTION

### <First Embodiment>

A first embodiment of the information processing system will be described below. In the first embodiment, an anterior chamber depth of a subject (a distance from the cornea to the crystalline lens) is estimated from an image of an eye of the subject. A shallow anterior chamber depth increases the risk of an acute glaucoma attack or a glaucoma attack associated with pupil dilation. Since the anterior chamber depth can be estimated from the captured image of the subject's eye, the risk of such a glaucoma attack can also be assessed.

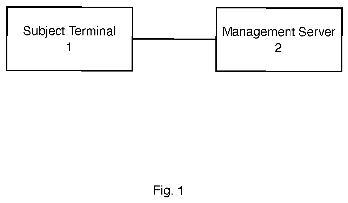

FIG. 1 illustrates an example of an overall configuration of the information processing system. The information processing system according to the present embodiment includes a management server 2. The management server 2 is communicably connected to a subject terminal 1 via a communication network. The communication network is, for example, the Internet, and may be constructed using a public telephone network, a mobile telephone network, a wireless communication channel, Ethernet (registered trademark), or the like.

The subject terminal 1 is a computer operated by the subject. The subject terminal 1 may be, for example, a smartphone, a tablet computer, or a personal computer. The subject terminal 1 includes a camera (not illustrated) and is capable of capturing an image of the subject's face, particularly the subject's eye. The subject terminal 1 may be operated by an examination assistant instead of by the subject.

The management server 2 may be a general-purpose computer such as a workstation or a personal computer, or may be logically implemented by cloud computing.

### <Subject Terminal 1>

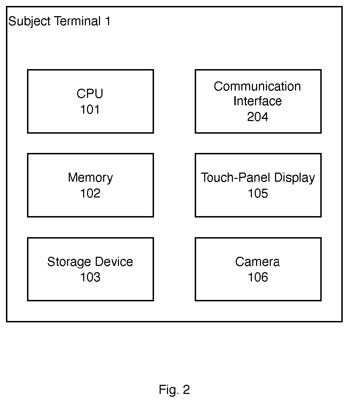

FIG. 2 illustrates an example of a hardware configuration of the subject terminal 1. The illustrated configuration is merely an example, and the subject terminal 1 may have another configuration. The subject terminal 1 includes a CPU 101, a memory 102, a storage device 103, a communication interface 104, a touch-panel display 105, and a camera 106. The storage device 103 stores various data and programs and may be, for example, a hard disk drive, a solid-state drive, or flash memory. The communication interface 104 is an interface for connection to the communication network, and may be, for example, an adapter for connection to Ethernet (registered trademark), a modem for connection to a public telephone network, a wireless communication device, or a Universal Serial Bus (USB) connector or an RS-232C connector for serial communication. The touch-panel display 105 is an input/output interface that displays an image on a screen and detects a position touched on the screen. The camera 106 can acquire a captured image. Each functional unit of the subject terminal 1 described below is implemented by the CPU 101 reading a program stored in the storage device 103 into the memory 102 and executing the program. Each storage unit of the subject terminal 1 is implemented as a part of a storage area provided by the memory 102 and the storage device 103.

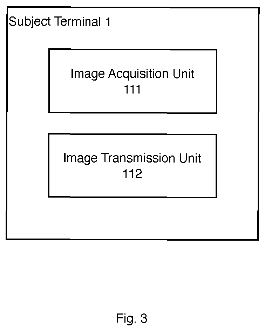

FIG. 3 illustrates an example of a software configuration of the subject terminal 1. The subject terminal 1 includes an image acquisition unit 111 and an image transmission unit 112.

The image acquisition unit 111 acquires an image captured by the camera 106 (hereinafter referred to as a captured image). The image acquisition unit 111 can control the camera 106 by a known method to acquire the captured image. When causing the camera 106 to capture an image, the image acquisition unit 111 may output, for example, a message instructing the subject to position the subject's eye for imaging. The image acquisition unit 111 may acquire an image in response to activation by the subject, or may acquire a captured image in response to receipt of a message from the management server 2 instructing image capture.

The image acquisition unit 111 may accept designation of a previously captured image. For example, the image acquisition unit 111 may accept designation of an image of the subject's eye from among images registered in an image storage unit such as a camera roll. Alternatively, in response to designation by the subject, the image acquisition unit 111 may read a captured image from a storage device in which the file is stored, including a storage medium included in or connected to the subject terminal 1 or a storage device included in an external server.

It is assumed that the captured image includes the subject's eye. The captured image may depict the subject's face or only a region around the subject's eye. The image acquisition unit 111 may determine whether an eye is included in the captured image. For example, the image acquisition unit 111 may input the captured image to a model for detecting an eye and determine whether an eye is included based on whether an eye can be detected from the captured image. If no eye is included, the image acquisition unit 111 may output a message instructing the subject to recapture the image and may again acquire an image captured by the camera 106 or a previously captured image.

The image transmission unit 112 transmits the captured image acquired by the image acquisition unit 111 to the management server 2.

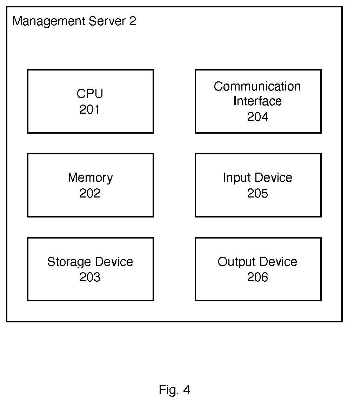

FIG. 4 illustrates an example of a hardware configuration of the management server 2. The illustrated configuration is merely an example, and another configuration may be used. The computer includes a CPU 201, a memory 202, a storage device 203, a communication interface 204, an input device 205, and an output device 206. The storage device 203 stores various data and programs and may be, for example, a hard disk drive, a solid-state drive, or flash memory. The communication interface 204 is an interface for connection to the communication network, and may be, for example, an adapter for connection to Ethernet (registered trademark), a modem for connection to a public telephone network, a wireless communication device, or a USB connector or an RS-232C connector for serial communication. The input device 205 inputs data and may be, for example, a keyboard, a mouse, a touch panel, a button, or a microphone. The output device 206 outputs data and may be, for example, a display, a printer, or a speaker. Each functional unit of the management server 2 described below is implemented by the CPU 201 reading a program stored in the storage device 203 into the memory 202 and executing the program. Each storage unit of the management server 2 is implemented as a part of a storage area provided by the memory 202 and the storage device 203.

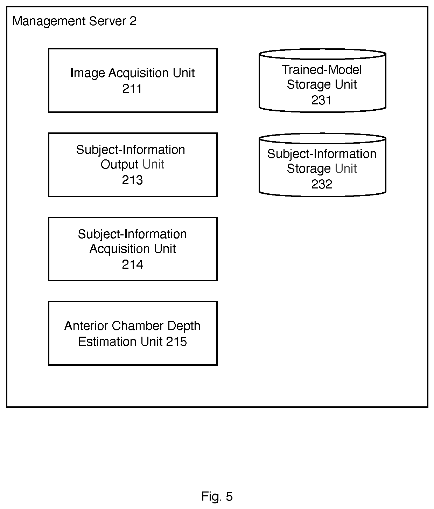

FIG. 5 illustrates an example of a software configuration of the management server 2. The management server 2 includes a trained-model storage unit 231, a subject-information storage unit 232, an image acquisition unit 211, a subject-information output unit 213, a subject-information acquisition unit 214, and an anterior chamber depth estimation unit 215.

The trained-model storage unit 231 stores a trained model for estimating an anterior chamber depth of a subject. The trained model stored in the trained-model storage unit 231 may be generated, for example, by machine learning using images of eyes and anterior chamber depths as training data. The trained model may also be generated by machine learning using images of eyes, anterior chamber depths, and one or both of the subject's age and sex as training data. The trained model may be updated by machine learning based on feedback including a captured image of the subject's eye and the subject's anterior chamber depth, for example, an anterior chamber depth measured by another device and received from the subject terminal 1. The trained-model storage unit 231 may be provided in an external server rather than in the management server 2, and the trained model may be used through an API provided by the external server.

The subject-information storage unit 232 stores subject information relating to the subject. The subject information may include one or both of the subject's age and sex. The subject information may further include any attribute of the subject, such as name and address. An estimated value of the anterior chamber depth estimated by the anterior chamber depth estimation unit 215 described below may also be set in the subject information.

The image acquisition unit 211 acquires a captured image of the subject's eye. The image acquisition unit 211 may receive data of a captured image transmitted from the subject terminal 1. The image acquisition unit 211 may transmit to the subject terminal 1 a message instructing capture of an image of the subject's eye. In response to the message, the subject terminal 1 may capture an image, and the image acquisition unit 211 may receive the captured image of the eye from the subject terminal 1.

The subject-information acquisition unit 214 acquires subject information. The subject information acquired by the subject-information acquisition unit 214 need not include an anterior chamber depth. For example, the subject-information acquisition unit 214 may receive, from the subject terminal 1, values of items included in the subject information, such as the subject's name, address, age, and sex, and register the values as subject information in the subject-information storage unit 232. The subject information may also include a refractive value of the subject's eye, such as spherical power, cylindrical power, spherical equivalent, or cylinder axis.

The anterior chamber depth estimation unit 215 estimates an anterior chamber depth of the subject. The anterior chamber depth estimation unit 215 may input the acquired captured image to the trained model stored in the trained-model storage unit 231, thereby estimating the anterior chamber depth of the subject.

The anterior chamber depth estimation unit 215 may input, together with the acquired captured image, at least one item of the subject information stored in the subject-information storage unit 232 to the trained model stored in the trained-model storage unit 231, thereby estimating the anterior chamber depth of the subject. For example, the anterior chamber depth estimation unit 215 may input the acquired captured image and one or both of the subject's age and sex to the trained model to estimate the anterior chamber depth. The anterior chamber depth estimation unit 215 may also input the acquired captured image and the refractive value in the subject information to the trained model to estimate the anterior chamber depth.

The subject-information output unit 213 outputs information relating to the subject (hereinafter referred to as subject information). The subject information may include information identifying the subject and the estimated anterior chamber depth of the subject. The subject-information output unit 213 may transmit the subject information to the subject terminal 1, output the subject information to an output device such as a display, or transmit the subject information to a terminal (not illustrated) of a healthcare professional such as an ophthalmologist.

### <Operation>

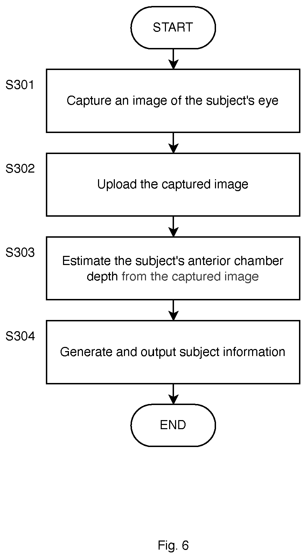

FIG. 6 is a diagram for explaining an operation of the information processing system.

The subject operates the subject terminal 1 to capture an image of the subject's own eye (S301), and the subject terminal 1 transmits the captured image to the management server 2 (S302).

The management server 2 inputs the captured image received from the subject terminal 1 to the trained model stored in the trained-model storage unit 231 to estimate the anterior chamber depth of the subject (S303). The management server 2 may generate and output subject information including the anterior chamber depth of the subject (S304).

As described above, the information processing system according to the first embodiment can estimate the anterior chamber depth of the subject from the captured image of the subject's eye. The estimated anterior chamber depth can be used to assess the risk of glaucoma.

The management server 2 may further include a glaucoma-attack risk determination unit configured to determine, based on the estimated anterior chamber depth, the risk of an acute glaucoma attack or a glaucoma attack associated with pupil dilation. In this case, the subject-information output unit 213 may output the glaucoma risk together with, or in place of, the estimated anterior chamber depth.

### <Disclosure Items>

The present disclosure also includes the following configurations.

### [Item 1]

An information processing system comprising:
an image acquisition unit configured to acquire a captured image of an eye of a subject; and
an anterior chamber depth estimation unit configured to input the acquired captured image to a trained model trained by machine learning using images of eyes and anterior chamber depths as training data, thereby estimating an anterior chamber depth of the subject.

### [Item 2]

The information processing system according to Item 1, further comprising:
a subject-information storage unit configured to store one or both of the subject's age and sex,
wherein the trained model is trained by machine learning using the images of eyes, the anterior chamber depths, and one or both of the subject's age and sex as training data, and
the anterior chamber depth estimation unit inputs the acquired captured image and one or both of the subject's age and sex stored in the subject-information storage unit to the trained model, thereby estimating the anterior chamber depth of the subject.

### [Item 3]

The information processing system according to Item 1, further comprising:
a subject-information storage unit configured to store a refractive value of the eye of the subject,
wherein the trained model is trained by machine learning using the images of eyes, the anterior chamber depths, and the refractive values as training data, and
the anterior chamber depth estimation unit inputs the acquired captured image and the refractive value stored in the subject-information storage unit to the trained model, thereby estimating the anterior chamber depth of the subject.

### [Item 4]

An information processing method executed by a computer, the method comprising:
acquiring a captured image of an eye of a subject; and
inputting the acquired captured image to a trained model trained by machine learning using images of eyes and anterior chamber depths as training data, thereby estimating an anterior chamber depth of the subject.

### [Item 5]

A program causing a computer to execute:
acquiring a captured image of an eye of a subject; and
inputting the acquired captured image to a trained model trained by machine learning using images of eyes and anterior chamber depths as training data, thereby estimating an anterior chamber depth of the subject.

### <Second Embodiment>

A second embodiment of the information processing system will next be described. In the second embodiment, cataract severity of a subject is estimated from an image of an eye of the subject.

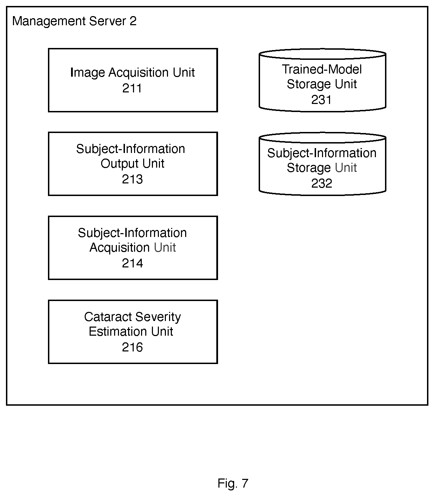

FIG. 7 illustrates an example of a software configuration of the management server 2 according to the second embodiment. In the second embodiment, the management server 2 includes a trained-model storage unit 231, a subject-information storage unit 232, an image acquisition unit 211, a subject-information output unit 213, a subject-information acquisition unit 214, and a cataract severity estimation unit 216. Compared with the configuration of the management server 2 according to the first embodiment, the anterior chamber depth estimation unit 215 is omitted and the cataract severity estimation unit 216 is added. The management server 2 according to the second embodiment may also include the anterior chamber depth estimation unit 215. Differences from the first embodiment will primarily be described below.

The trained model stored in the trained-model storage unit 231 according to the second embodiment may be generated by machine learning using images of eyes and cataract severity as training data.

The trained model may also be generated by machine learning using images of eyes and anterior chamber depths as training data.

The trained model may also be generated by machine learning using images of eyes, anterior chamber depths, and one or both of the subject's age and sex as training data. An estimated value of the cataract severity estimated by the cataract severity estimation unit 216 described below may also be set in the subject information stored in the subject-information storage unit 232.

The subject information output by the subject-information output unit 213 includes the estimated cataract severity.

The subject-information acquisition unit 214 acquires subject information. The acquired subject information need not include cataract severity. For example, the subject-information acquisition unit 214 may receive values of items such as the subject's name, address, age, and sex from the subject terminal 1 and register the values in the subject-information storage unit 232. The subject information may also include a refractive value of the subject's eye, such as spherical power, cylindrical power, spherical equivalent, or cylinder axis.

The cataract severity estimation unit 216 estimates cataract severity (lens opacity level) of the subject. The cataract severity estimation unit 216 may input the acquired captured image to the trained model stored in the trained-model storage unit 231, thereby estimating the cataract severity of the subject.

The cataract severity estimation unit 216 may input, together with the acquired captured image, at least one item of the subject information stored in the subject-information storage unit 232 to the trained model, thereby estimating the cataract severity. For example, the cataract severity estimation unit 216 may input the acquired captured image and one or both of the subject's age and sex to the trained model to estimate the cataract severity.

As described above, the information processing system according to the second embodiment can estimate the cataract severity of the subject from the captured image of the subject's eye.

The disclosure according to the second embodiment may include the following configurations.

### [Item 1]

An information processing system comprising:
an image acquisition unit configured to acquire a captured image of an eye of a subject; and
a cataract severity estimation unit configured to input the acquired captured image to a trained model trained by machine learning using images of eyes and cataract severity as training data, thereby estimating cataract severity of the subject.

### [Item 2]

The information processing system according to Item 1, further comprising:
a subject-information storage unit configured to store one or both of the subject's age and sex,
wherein the trained model is trained by machine learning using the images of eyes, the cataract severity, and one or both of the subject's age and sex as training data, and
the cataract severity estimation unit inputs the acquired captured image and one or both of the subject's age and sex stored in the subject-information storage unit to the trained model, thereby estimating the cataract severity of the subject.

### [Item 3]

An information processing method executed by a computer, the method comprising:
acquiring a captured image of an eye of a subject; and
inputting the acquired captured image to a trained model trained by machine learning using images of eyes and cataract severity as training data, thereby estimating cataract severity of the subject.

### [Item 4]

A program causing a computer to execute:
acquiring a captured image of an eye of a subject; and
inputting the acquired captured image to a trained model trained by machine learning using images of eyes and cataract severity as training data, thereby estimating cataract severity of the subject.

### <Third Embodiment>

A third embodiment of the information processing system will next be described. In the third embodiment, a corneal endothelial cell density of a subject is estimated from an image of an eye of the subject.

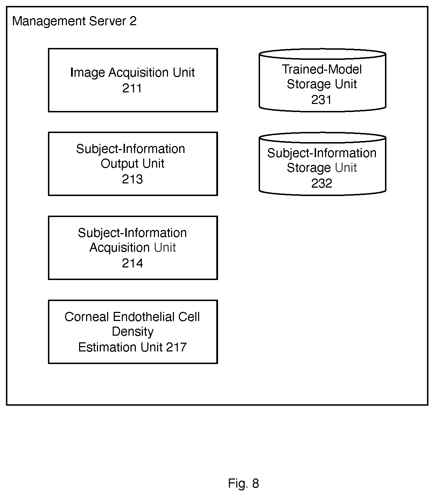

FIG. 8 illustrates an example of a software configuration of the management server 2 according to the third embodiment. In the third embodiment, the management server 2 includes a trained-model storage unit 231, a subject-information storage unit 232, an image acquisition unit 211, a subject-information output unit 213, a subject-information acquisition unit 214, and a corneal endothelial cell density estimation unit 217. Compared with the first embodiment, the anterior chamber depth estimation unit 215 is omitted and the corneal endothelial cell density estimation unit 217 is added. The management server 2 according to the third embodiment may also include the anterior chamber depth estimation unit 215 and/or the cataract severity estimation unit 216.

The trained model stored in the trained-model storage unit 231 according to the third embodiment may be generated by machine learning using images of eyes and corneal endothelial cell densities as training data. The trained model may also be generated by machine learning using images of eyes, corneal endothelial cell densities, and one or both of the subject's age and sex as training data.

An estimated value of the corneal endothelial cell density estimated by the corneal endothelial cell density estimation unit 217 described below may also be set in the subject information stored in the subject-information storage unit 232.

The subject information output by the subject-information output unit 213 includes the estimated corneal endothelial cell density.

The subject-information acquisition unit 214 acquires subject information. The acquired subject information need not include a corneal endothelial cell density. For example, the subject-information acquisition unit 214 may receive values of items such as the subject's name, address, age, and sex from the subject terminal 1 and register the values in the subject-information storage unit 232. The subject information may also include a refractive value of the subject's eye, such as spherical power, cylindrical power, spherical equivalent, or cylinder axis.

The corneal endothelial cell density estimation unit 217 estimates a corneal endothelial cell density of the subject. The corneal endothelial cell density estimation unit 217 may input the acquired captured image to the trained model stored in the trained-model storage unit 231, thereby estimating the corneal endothelial cell density of the subject.

The corneal endothelial cell density estimation unit 217 may input, together with the acquired captured image, at least one item of the subject information stored in the subject-information storage unit 232 to the trained model, thereby estimating the corneal endothelial cell density. For example, the corneal endothelial cell density estimation unit 217 may input the acquired captured image and one or both of the subject's age and sex to the trained model to estimate the corneal endothelial cell density.

As described above, the information processing system according to the third embodiment can estimate the corneal endothelial cell density of the subject from the captured image of the subject's eye.

Information indicating whether the subject uses contact lenses may be managed as part of the subject information, and a message transmission unit may be provided to transmit, to a subject who uses contact lenses, a message recommending an examination of corneal endothelial cell density. The message transmission unit may transmit the message periodically, or after a predetermined period has elapsed following user registration or a previous corneal endothelial cell density examination, thereby encouraging periodic monitoring of corneal endothelial cell density.

The disclosure according to the third embodiment may include the following configurations.

### [Item 1]

An information processing system comprising:
an image acquisition unit configured to acquire a captured image of an eye of a subject; and
a corneal endothelial cell density estimation unit configured to input the acquired captured image to a trained model trained by machine learning using images of eyes and corneal endothelial cell densities as training data, thereby estimating a corneal endothelial cell density of the subject.

### [Item 2]

The information processing system according to Item 1, further comprising:
a subject-information storage unit configured to store one or both of the subject's age and sex,
wherein the trained model is trained by machine learning using the images of eyes, the corneal endothelial cell densities, and one or both of the subject's age and sex as training data, and
the corneal endothelial cell density estimation unit inputs the acquired captured image and one or both of the subject's age and sex stored in the subject-information storage unit to the trained model, thereby estimating the corneal endothelial cell density of the subject.

### [Item 3]

An information processing method executed by a computer, the method comprising:
acquiring a captured image of an eye of a subject; and
inputting the acquired captured image to a trained model trained by machine learning using images of eyes and corneal endothelial cell densities as training data, thereby estimating a corneal endothelial cell density of the subject.

### [Item 4]

A program causing a computer to execute:
acquiring a captured image of an eye of a subject; and
inputting the acquired captured image to a trained model trained by machine learning using images of eyes and corneal endothelial cell densities as training data, thereby estimating a corneal endothelial cell density of the subject.

Although the embodiments have been described above, the embodiments are provided to facilitate understanding of the present invention and are not intended to limit the present invention. The present invention may be modified or improved without departing from the spirit thereof, and includes equivalents thereof.

### Description of the Reference Numerals

1 Subject terminal
2 Management server

## Claims

1. An information processing system comprising:
an image acquisition unit configured to acquire a captured image of an eye of a subject; and
an anterior chamber depth estimation unit configured to input the acquired captured image to a trained model trained by machine learning using images of eyes and anterior chamber depths as training data, thereby estimating an anterior chamber depth of the subject.

2. The information processing system according to claim 1, further comprising:
a subject-information storage unit configured to store one or both of the subject's age and sex, wherein the trained model is trained by machine learning using the images of eyes, the anterior chamber depths, and one or both of the subject's age and sex as training data, and
the anterior chamber depth estimation unit inputs the acquired captured image and one or both of the subject's age and sex stored in the subject-information storage unit to the trained model, thereby estimating the anterior chamber depth of the subject.

3. The information processing system according to claim 1, further comprising:
a subject-information storage unit configured to store a refractive value of the eye of the subject,
wherein the trained model is trained by machine learning using the images of eyes, the anterior chamber depths, and the refractive values as training data, and
the anterior chamber depth estimation unit inputs the acquired captured image and the refractive value stored in the subject-information storage unit to the trained model,
thereby estimating the anterior chamber depth of the subject.

4. An information processing method executed by a computer, the method comprising:
acquiring a captured image of an eye of a subject; and
inputting the acquired captured image to a trained model trained by machine learning using images of eyes and anterior chamber depths as training data, thereby estimating an anterior chamber depth of the subject.

5. A program causing a computer to execute:
acquiring a captured image of an eye of a subject; and
inputting the acquired captured image to a trained model trained by machine learning using images of eyes and anterior chamber depths as training data, thereby estimating an anterior chamber depth of the subject.
